# EUROPEAN PATENT SPECIFICATION

(11) **EP 2 501 429 B1**
(45) Date of publication and mention of the grant of the patent: **09.01.2019**
(21) Application number: 10832003.7
(22) Date of filing: 09.11.2010
(51) Int. Cl.: A61M 25/02

(54) **TUBE HOLDER For SECURING CATHETERS TO A PATIENT**
TUBUSHALTER ZUR SICHERUNG VON KATHETERN IM KÖRPER EINES PATIENTEN
PORTE-TUBE DE FIXATION DE CATHÉTERS SUR UN PATIENT

(30) Priority: 18.11.2009 US 620844
(43) Date of publication of application: 26.09.2012
(73) Proprietor: Bioderm, Inc., Largo, FL 33773 (US)
(72) Inventor: KAY, Dennis M., Largo FL 33773 (US); UNDERWOOD, David W., Tampa FL 33609 (US); BABB, Steven J., Seminole FL 33777 (US)
(74) Representative: Murgitroyd & Company
(86) International application number: PCT/US2010/055935
(87) International publication number: WO 2011/062801

(56) References cited:
- WO-A2-2005/104776
- WO-A2-2007/028007
- US-A- 3 302 913
- US-A- 4 316 461
- US-A- 4 416 664
- US-A- 4 662 873
- US-A- 5 084 026
- US-A- 5 195 981
- US-A- 5 232 453
- US-A- 5 304 146
- US-A- 5 897 519
- US-A- 6 102 347
- US-A1- 2005 038 453
- US-A1- 2006 276 752
- US-A1- 2006 276 752
- US-A1- 2009 139 061
- US-A1- 2009 216 197

## Description

### BACKGROUND OF THE INVENTION

### Field of the Invention:

The present invention pertains to devices for securing elongate members, such as medical tubing, and more particularly, to devices utilizing a tube holder to secure catheters to a patient's body.

### Brief Discussion of the Related Art:

Medical catheters are used to transfer liquids and gases (fluids) to and/or from naturally or surgically created openings such as stoma, the oral cavity, the urethra or percutaneous central sites such as for venous catheters. Catheter safety and reliability depend on assuring patency of the lumen of the catheter and securement such that physical forces are not transmitted internally to the patient. Design and materials of a catheter securement device are needed so as to not cause injury to the patient, such as maceration, pressure ulcers, skin tears and infections for example.

Catheters commonly range in size from 1.9 French (approximately 0.627 mm) for neonatal applications through thoracostomy tubes up to 44 French (approximately 14.52 mm) and include many varieties of mid-size catheters (for example, intravenous, suprapubic, urinary drainage, etc.). To reduce caregiver training costs and improve catheter securement and insertion site dressing technique compliance, a safe and reliable catheter securement device is desirable which can be used for the entire range of commonly used catheters.

One of the disadvantages of prior art catheter securement devices (tube holders), see e.g. WO2005104776, WO2007028007 or US5304146, is that such catheter securement devices permit in-and-out motion or "pistoning" of catheters at the point of entry (insertion site) into a patient's body, and even slight in-and-out motion or "pistoning" can cause infection in that the sliding movement of a catheter with respect to an insertion site carries organisms (pathogens) through the epidermal barrier to cause such infections. Such sliding movement is not adequately prevented by medical tape and catheter holders or securement devices currently available.

The walls of catheters are typically subject to various physical forces which are potentially detrimental to the patency of the catheter lumen and may cause tissue injury to a patient. Polymeric materials used for catheter construction typically exhibit a high flexural modulus, softness (i.e. low durometer) and high modulus of elasticity resulting in catheters which are pliable but resist stretch. Conventional polymeric materials include silicone rubber and various thermoplastic elastomers such as polyisobutylene (latex), polyvinylchloride (PVC), fluorinated ethylene proprylene (FEP), polytetrafluoroethylene (PTFE) and polyurethane. Material properties which make catheters pliable also render the catheters susceptible to crushing such as at rigid pinch points in various prior art catheter securement devices. Kinking is another factor which can cause significant loss of catheter patency such as when a catheter is forced to bend excessively over a short distance. Catheters are also subject to twisting forces along the longitudinal axes thereof caused by the arrangement of other medical devices or patient movement. Catheters are also subject to tugging and leverage forces caused by various factors such as bending at the catheter insertion site into a body. Prior art catheter securement devices do not adequately protect against the above-mentioned disadvantages such that tissue damage can occur wherever a patient's skin is crushed or tom (ischemia) and also do not permit a controlled and stable angle of entry at the insertion site for a catheter to avoid near 90° entry of the catheter at the insertion site which can allow leverage forces to be transmitted through the catheter thereby causing crushing at the outboard interface and tearing at the inboard interface. Detrimental leverage forces also occur with the use of catheter securement devices having a swiveling gripping design.

### SUMMARY OF THE INVENTION

The present invention, as claimed in claim 1, provides a medical tubing arrangement (e.g. for catheters) positively gripping the tubing for avoiding the above-described disadvantages of the prior art relating to physical forces encountered in clinical practice for the normal range of catheter sizes.

Further, the present invention incorporates a tube holder providing standardized, positive gripping of catheters of various sizes with a support surface in the form of an elastic skin attachment/foundation pad capable of stretching over swollen tissue (edema or anasarca) while maintaining excellent adhesion over complex body surfaces or curvatures which can change continuously due to body movement or repositioning.

Further, the present invention provides a tube holder that firmly grips catheters of varying sizes to reduce "tug trauma", i.e. gradual or sudden breaking of the skin/adhesive bond encountered when semi-or fully-rigid securement devices do not stretch with natural skin movement which can cause an adhesive bond to shear.

Furthermore, a tube holder according to the present invention utilizes male and female straps with the female straps having a plurality of apertures therein for receiving locking members carried by a male strap such that, dependent upon the aperture into which locking members of the male strap are inserted, the tube holder can firmly grip catheters of varying sizes.

Furthermore, a tube holder includes a female strap having a plurality of apertures therein with the aperture closer to a base of the tube holder having a height less than the height of the other apertures thereby facilitating a gripping of small diameter tubes by flexible locking members carried by a male strap extending through the smaller aperture.

Furthermore, the present invention firmly grips various size tubes with first and second gripping members of a tube holder by configuring one of the gripping members to have an configuration defining an engagement portion to cooperate with an engagement portion of the other gripping member to securely hold the tubes with jaws in a "channel lock" manner.

In a further aspect, a tube holder according to the present invention utilizes a base with a raised curved or dome-like platform to form a shock-absorbing and isolating structure mounting male and female straps for gripping tubes.

Some of the advantages of the present invention over prior art tube holder/catheter securement arrangements include increased securement (gripping) of tubes, increased infection control, cooperation with skin physiology to reduce injuries, safety in use due to elimination of structures which could cause tissue cuts, maceration or necrosis, easier training of caregivers and use by caregivers due to standardization (i.e. single tube holder for various size catheters), protection of lumen patency across a wide range of catheter dimensions, materials and clinical applications, reduction of the potential for infection due to the ability to create an occlusive seal against pathogens and/or prevention of catheter dislodgement.

Other aspects and advantages of the present invention will become apparent from the following description of the invention taken in conjunction with the accompanying drawings, wherein like parts in each of the several figures are identified by the same reference characters.

### BRIEF DESCRIPTION OF THE DRAWINGS

Fig. 1 is a perspective view of a tube holder according to the present invention.
Figs. 2 and 3 are opposite side views, respectively, of the tube holder of Fig. 1.
Figs. 4 and 5 are opposite end views, respectively, of the tube holder of Fig. 1.
Fig. 6 is a broken perspective view showing the tube holder of Fig. 1 secured to a support surface in the form of an adhesive layer for attachment to the skin and holding a small diameter catheter.
Fig. 6A is a section taken along line 6A-6A of Fig. 6.
Fig. 7 is a perspective view of the tube holder of Fig. 1 attached to a patient's body via a support surface in the form of a strap and holding a catheter larger in diameter than the catheter in Fig. 6.
Fig. 8 is an end view showing the tube holder of Fig. 1 gripping a small tube.
Fig. 9 is an end view showing the tube holder of Fig. 1 gripping an intermediate size tube.
Fig. 10 is an end view showing the tube holder of Fig. 1 gripping a large tube.

### DETAILED DESCRIPTION OF THE INVENTION

As used herein, the term "tube" means an elongate member (i.e. a member having a length greater than its diameter/width) regardless of the cross-sectional configuration thereof (e.g. round, circular, oval, curved, three or more sided, polygonal or the like). The "tube" is hollow or partially hollow or can be formed of a slotted sleeve, such as a catheter support sleeve. As used herein, the term "body" means a corpus, such as of an animal or a human, or a part thereof such as skin or an appendage.

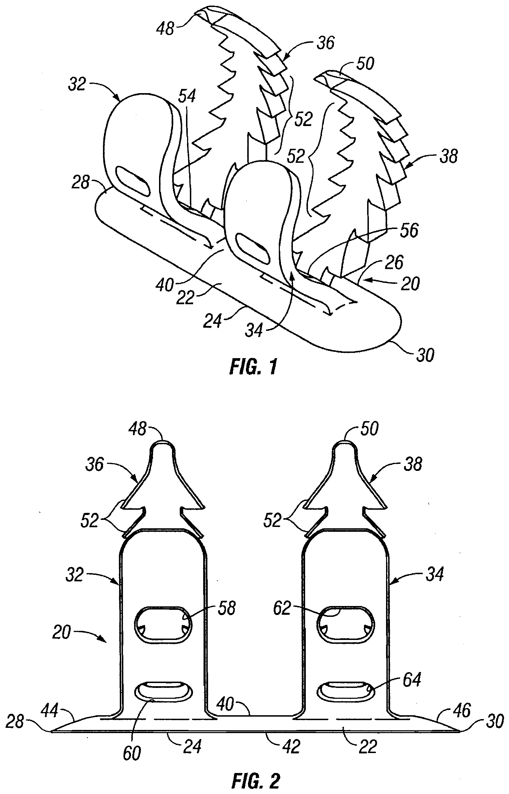

A tube holder 20 according to the present invention, as shown in Figs. 1-5, includes an elongated base 22 having opposing lateral sides or edges 24 and 26 and opposing longitudinal ends or edges 28 and 30 such that the spacing between sides 24 and 26 defines a width for the base and the spacing between ends 28 and 30 defines a length for the base with the length being greater than the width. Extending substantially transversely from the base are gripping members in the form of female straps 32 and 34 and gripping members in the form of male straps 36 and 38 in lateral alignment with female straps 32 and 34, respectively. The female straps are longitudinally spaced along the base, and the male straps are similarly longitudinally spaced along the base such that the male straps 36 and 38 are in opposed alignment with the female straps 32 and 34, respectively, forming pairs of gripping members 32,36 and 34,38.

The base 22 has a central domed platform 40 raised above a substantially flat bottom surface 42 to form a shock absorbing and isolating structure upon which the male and female straps are mounted. The flat bottom surface 42 of the base 22 extends widthwise entirely across the width of the base, and the raised platform 40 has a domed upper surface spaced from the bottom surface 42 with the width of the platform being less than the width of the base. The male and female straps extend arcuately from the lateral sides of the platform to curve laterally and upwardly away from the base. The longitudinal ends 28 and 30 of the base have surfaces 44 and 46, respectively, sloping from the upper surface of the platform 40 to the bottom surface 42 of the base at an angle on the order of 21° to accommodate the preferred angle of entry for catheters into a patient's body thereby reducing upward forces or leverage that would occur if the base had transversely extending (square) longitudinal ends. The sloping surfaces 44 and 46 support catheter tubes as the tubes extend toward the body of a patient from the platform 40.

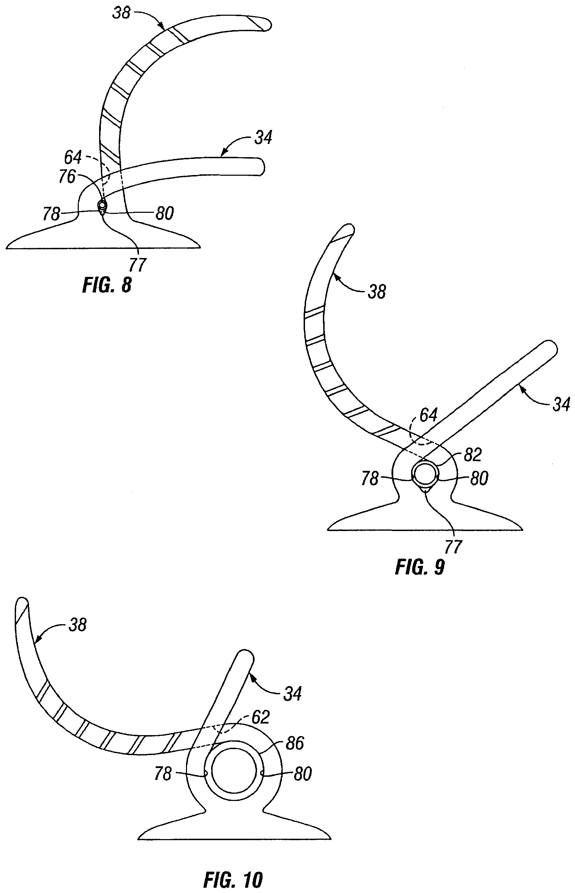

The male straps 36 and 38 have tapered free ends 48 and 50, respectively; and, a series of flared, arrow-shaped, flexible locking members or detents 52 are carried along the lengths of the male straps of a size to pass through the apertures in the female straps in a flexed condition. More particularly, the arrow-like shapes of the flexible locking members 52 allow the flanges thereof to flex while passing through an aperture and then return to an unflexed position to lock the male straps within the female straps. The male straps 36 and 38 have fixed ends 54 and 56, respectively, coupled with the base 22 along a lateral side of the domed platform 40, and the flexible locking members 52 of each strap are carried along a flexible body of the strap extending between the fixed end and the free end with the flexible bodies of the male straps being arcuate in configuration to define engagement portions adjacent the fixed ends as illustrated in Figs. 8, 9 and 10.

The female straps each have a plurality of apertures therein spaced along the length thereof. As shown in Fig. 2, female straps 32 and 34 each have at least two apertures 58,60 in female strap 32 and 62,64 in female strap 34. The lower apertures 60 and 64 looking at Fig. 2 are closer to the base 22 than the upper apertures 58 and 62, and the apertures are sized such that the heights of apertures 60 and 64 are less than the heights of apertures 58 and 62, respectively. The lower heights of the apertures closer to the base provide secure gripping of smaller diameter tubes and also minimize the opportunity for a small diameter tube to be caught therein or pass therethrough.

The male and female straps join each other at their fixed ends providing a trough with a bottom for receiving and gripping tubes. The base, the gripping members/male and female straps are unitarily, integrally formed of a one-piece soft, low durometer elastomer having a hardness less than or equal to 30 Shore A.

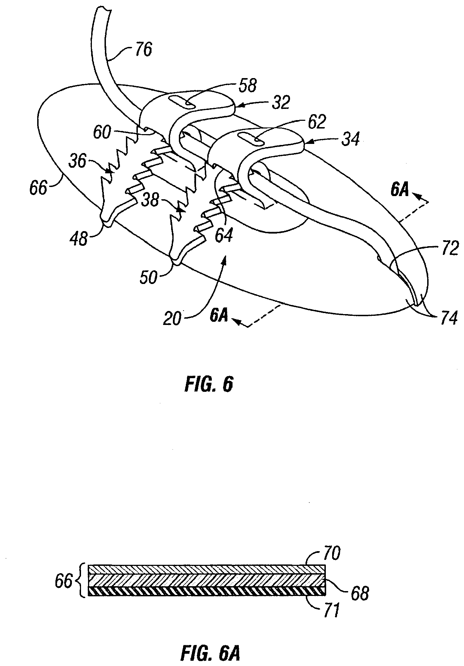

As shown in Fig. 6, the tube holder 20 includes a support surface or member 66 in the form of an adhesive foundation pad for securement to a patient's body with the base 22 mounted on the support member. As shown in Fig. 6A, the support member 66 is formed of an adhesive layer 68, such as hydrocolloid, bonded to an elastomeric film carrier 70, such as polyurethane, to provide ergonomic and skin-compatible features including moisture balance and elasticity similar to skin, preferably at least 250% elongation, most preferably 300% elongation. The ergonomics result in gradual and uniform transfer of stresses originated from a catheter, and the hydrocolloid on the elastomeric film also provides for extended wear, is hypoallergenic and creates a firm hold even in the presence of perspiration. The adhesive and film combination is preferably transparent or semi-transparent to allow additional infection control by permitting visual detection of any potential underlying skin irritation or infection. The film 70 can be made of polyurethane or any other suitable elastomeric material and as shown, the film 70 is disposed between the bottom surface 42 of base 22 and the layer of hydrocolloid 68. The adhesive layer/pad 66 optimally has an elliptical shape, preferably with a major axis length of twice the length of the base 22 and a major to minor axis ratio of 1.5 to assist in assuring natural movement with the skin. An elliptical shape provides a geometry to assure optimal confirmation to contours of a patient's body which may change shape in multiple planes. The bottom of the adhesive layer 68 is covered with a siliconized release paper 71 which can be plow-folded to facilitate removal thereof prior to placement of the adhesive layer on a patient's skin.

When the tube holder 20 is utilized adjacent a catheter insertion site, the adhesive pad can have a slit or slot 72 therein such that a catheter can extend through the slot as shown in Fig. 6. The adhesive pad adjacent the slot can be used to cover entry of the catheter into the insertion site. When the slot is used, the catheter is inserted at the base of the slot, and the free ends of the ellipse of the adhesive pad which are created by the slot form flaps shown at 74 which overlap. The flaps provide a fully occlusive seal around the catheter which minimizes infection and adds to the positive grip of the catheter thus preventing in-and-out micromotion or pistoning of the catheter at the insertion site which otherwise could lead to distribution of bacterial films and various pathogens. Additionally, the overlapping arrangement of the flaps 74 provides a robust, yet flexible, occlusive seal and protects the skin surface from exposure to fluids or caustic material (e.g. gastric fluids) and further assures a controlled and stable angle of entry of the catheter at the skin/catheter interface to prevent tissue damage through either ischemia (tearing) and/or crushing. The slotted configuration of the adhesive foundation pad 66 coupled with the flexible, soft nature of the base and gripping members enhances the robustness of the occlusive seal with the catheter thereby resisting disruption of the seal. In use, the free ends or flaps of the foundation pad adjacent the slot are wrapped alternately around the catheter such that the hydrocolloid adhesive seals to both the skin of the patient and the foundation pad surfaces to provide the highly occlusive seal against pathogens and guard against in-and-out micromotions that are common with catheter holding devices of the prior art. Since the flaps are integral with the flexible foundation pad and encircle the catheter, the catheter is maintained in proper position at all points from the securement of the tube holder to the skin to the point of catheter insertion into the patient's body.

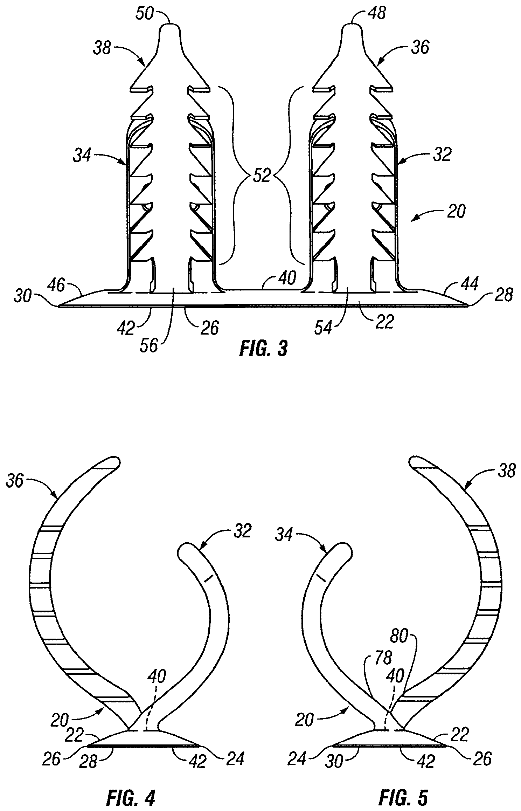

A small diameter tube 76 is shown in Fig. 6 being gripped by the female straps 32 and 34 and the male straps 36 and 38, the small diameter tube being, for example a 1.9 Fr catheter. The small diameter tube 76 is gripped by passing the free ends 48 and 50 of the male straps 36 and 38, respectively, through the lower, smaller size apertures 60 and 64 in the female straps 32 and 34, respectively, thereby securely gripping the tube 68 and limiting the opportunity for the tube to be drawn through the aperture along with the male strap. As shown in Fig. 8, the small diameter tube 76 is gripped in a trough or space 77 between a female strap 34 and a male strap 38 defined by engagement portions 78 and 80, respectively of female strap 34 and male strap 38. The space between the engagement portions does not conform completely to the configuration of the tube 76 such that the male and female gripping members/straps provide a channel jaw, pliers type arrangement due to the configurations and thicknesses of the lower segments of the male and female gripping members. That is, as best shown in Figs. 4 and 5, the thickness of the lower portion of each male member cooperates with the shape of each female gripping member to create a channel-like gripping arrangement therebetween.

Fig. 9 illustrates the securement of an intermediate size tube 82 in space 77 with the male gripping member 38 passing through the lower aperture 64 such that channel lock pliers-type gripping is created by the jaws formed by the engagement portions 78 and 80 of female and male gripping members 34 and 38, respectively.

Fig. 10 illustrates the securement of a large size tube 86 with the male gripping member 38 passing through the upper aperture 62 of the female gripping member to grip the tube with a channel lock plier-gripping arrangement but with the jaws or engagement portions of the gripping members having a configuration more closely approximating the outer configuration of the tube 86 such that engagement portion 78 of female gripping member 34 is arcuate as is the engagement portion 84 of male gripping member 38.

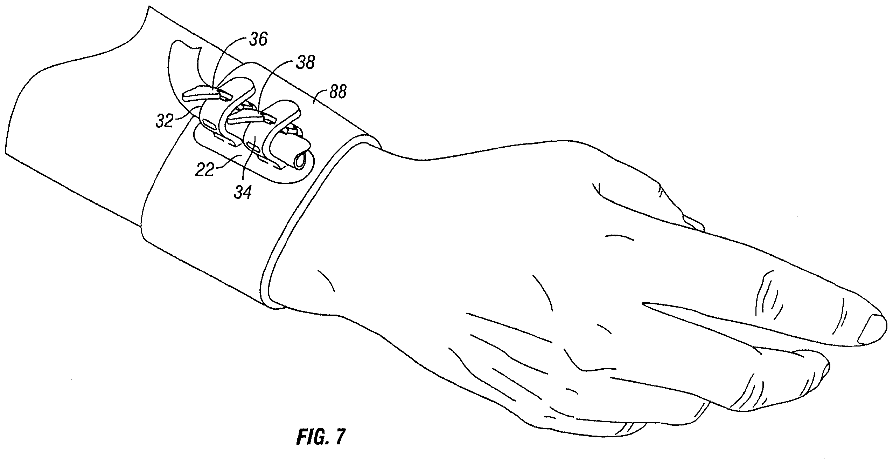

Fig. 7 illustrates a tube holder having a base and gripping members as shown in Figs. 1-5 but where the support surface or foundation pad is formed by a strap 88 surrounding a limb of a patient. With the base 22 being secured, by adhesive, to the strap, the use of a strap permits the tube holder to be positioned at various locations on a patient's body as well as on environmental surfaces adjacent a patient. The ends of the strap can be fastened via hook and loop type fasteners (Velcro) or other suitable fasteners such as snaps, buckles and the like or the strap can be elastic with no requirement for fastening of the ends together. Accordingly, the tube holder shown in Fig. 7 is reusable in nature and can be attached to various structures as desired.

The narrowed tips of the male gripping members eases insertion of the male members into a chosen aperture in the female gripping member even while the caregiver is wearing gloves. The thickness of the base and the configuration of the raised platform of the base reduces warping when the tube holder is subjected to stress as a gripping member/strap is pulled tight after insertion through an aperture in an opposing gripping member and further prevents transmission of the stress on the male strap to the patient's skin. The use of an adhesive layer such as hydrocolloid allows the support surface to act as a wound dressing as well as providing securement for the tube holder and further permits incorporation therein of antimicrobial agents to provide an antimicrobial catheter insertion site dressing. As previously noted, the thickening of the male straps near the base and the curvature of the male straps allow the male straps to come over a tube to be gripped to the female strap like channel lock jaws thus holding small (tiny) catheters. By forming the base and gripping members of the tube holder of a low durometer (less than 30 Shore A and as low as 3 Shore A elastomer), high friction elastomers can be used that grip tubes without kinking and transfer compound twisting and bending forces are transferred uniformly to a patient's skin in that the components of the tube holder act together to transfer significant compound forces to the compound body shapes where the tube holder is applied. With the forces isolated as noted above, the tube holder provides improved and uniform gripping force on tubes while reducing the potential for dislodgement and inadvertent tear-out of the tubes. The sloping surfaces at the longitudinal ends of the base provide a controlled and stable angle of entry of the catheter at a skin-catheter interface thus resisting transmission of leverage forces of the catheter which otherwise might potentially cause tissue damage through ischemia (tearing) and crushing and prevent the catheter from twisting or rotating about the longitudinal axis of the catheter. The use of an adhesive foundation pad, particularly including a hydrocolloid, provides a support surface capable of flexing to accommodate body contours, stretching to match the physiological properties of a patient's skin including swelling from edema and anasarca, facilitates natural movement of a patient's subcutaneous structures with body movement, and assures the skin/adhesive interface remains intact. The gripping arrangement of the tube holder allows a catheter to be easily changed without removing the adhesive support surface from the skin by folding the arrow-shaped flanges of the male gripping members to allow the male gripping members to be pulled backwards through the apertures in the female gripping members while simultaneously providing a positive lock on a catheter such that the catheter cannot be pulled out of the tube holder inadvertently or by a patient using a single hand.

A method of securing a catheter to a patient's body in accordance with the present invention utilizing a tube holder as described above includes the steps of securing the support surface to the patient's body, positioning the catheter between at least one pair of the male and female gripping members in the trough defined therebetween by the engagement portions thereof, selecting one of the apertures in the female gripping member based on the size of the catheter, inserting the tapered free end of the male gripping member in the selected aperture and pulling the male gripping member through the selected aperture to grip the catheter therebetween, it being noted that when multiple pairs of male/female gripping members are utilized, the gripping members are tightened sequentially.

Inasmuch as the present invention is subject to many variations, modifications and changes in detail, it is intended that the examples discussed above or shown in the accompanying drawings be interpreted as illustrative only and not be taken in a limiting sense.

## Claims

1. A tube holder (20) comprising
a support surface (66) for securement to a patient's skin including a hydrocolloid adhesive layer (68) bonded to an elastomeric film (70) to provide skin compatible features including an elasticity similar to human skin;
a base (22) mounted on said support surface, said base having a length
and a width; and
wherein said base has a flat bottom surface (42) extending widthwise entirely across said width of said base and a raised platform (40) having an upper surface spaced from said bottom surface and extending widthwise not entirely across a portion of said base such that said platform has a width less than said width of said base,
a first female strap extending substantially transversely from said base,
said first female strap (32) having a length extending from a fixed end carried on a side of said base to a free end and a plurality of apertures (58, 60) therein spaced along said length;
a second female strap (34) extending substantially transversely from said base at a position longitudinally spaced from said first female strap, said second female strap having a length extending from a fixed end carried on a side of said base to a free end and a plurality of apertures (62, 64) therein spaced along said length of said second female strap; and
wherein each of said fixed ends of said first and second female straps is carried on a side of said platform upper surface; and
a first male strap (36) extending substantially transversely from said base, said first male strap having a length extending from a fixed end (54) carried on a side of said base to a free end and carrying a plurality of flexible locking members (52) along said length of said first male strap, said locking members being of a size to pass through said plurality of apertures in said first female strap in a flexed condition; and
a second male strap (38) extending substantially transversely from said base at a position longitudinally spaced from said first male strap, said second male strap having a length extending from a fixed end (56) carried on a side of said base to a free end and carrying a plurality of flexible locking members (52) along said length of said second male strap, said locking members being of a size to pass through said plurality of apertures in said second female strap in a flexed condition; and
wherein the first and second male strap fixed ends are coupled with the base along a lateral side of said platform upper surface (40),
said first female and male straps being flexible to move toward each other and said second female and male straps being flexible to move toward each other whereby said locking members carried on said first and second male straps can be inserted in selected ones of said apertures in said first and second female straps, respectively, and pulled to grip a tube and hold the tube along said length of said base,
said base, said first and second female straps and said first and
second male straps being unitarily, integrally formed together of a one-piece soft, low durometer elastomer having a hardness less than or equal to 30 Shore A; and
wherein said first and second female straps and said first and
second male straps join each other at their fixed ends providing a trough (77) with a bottom for receiving and gripping tubes; and
wherein each of said fixed ends of said first and second male straps is carried on a side of said platform upper surface at a position laterally opposite one of said first or second female straps and each of said first and second male straps has a free end spaced from said platform upper surface and an arcuate body between said fixed end and said free end to form a tube gripping curvature when said free ends of said first and second male straps are each received in one of said apertures in the opposing one of said first or second female straps.

2. A tube holder as recited in claim 1 wherein said upper surface of said raised platform has a domed configuration.

3. A tube holder as recited in claim 2 wherein said base has end surfaces (44, 46) sloping from said upper surface of said platform to said bottom surface of said base.

4. A tube holder as recited in claim 1 wherein said hydrocolloid layer has an opening therein for receiving a medical catheter adjacent an insertion site.

5. A tube holder as recited in claim 4 wherein said opening is formed by a slot (72) defining flaps (74) to wrap around the medical catheter in an overlapping arrangement.

6. A tube holder as recited in claim 1 wherein said apertures in one of said female straps include at least two apertures therein spaced along said length, one of said apertures (60, 64) being closer to said base than the other (58, 62) of said apertures, said closer aperture having a height less than the height of said other aperture.

## Patentansprüche

1. Eine Schlauchhalterung (20), beinhaltend:
eine Auflageoberfläche (66) zur Sicherung an der Haut eines Patienten, umfassend eine Hydrokolloid-Klebeschicht (68), die mit einer Elastomerfolie (70) verbunden ist, um hautverträgliche Eigenschaften bereitzustellen, umfassend eine Elastizität, die der menschlichen Haut ähnelt;
eine Basis (22), die auf der Auflageoberfläche angebracht ist, wobei die Basis eine Länge und eine Breite aufweist; und
wobei die Basis eine flache untere Oberfläche (42), die sich der Breite nach vollständig über die Breite der Basis erstreckt, und eine erhobene Plattform (40) mit einer oberen Oberfläche aufweist, die von der unteren Oberfläche beabstandet ist und sich der Breite nach nicht vollständig über einen Abschnitt der Basis erstreckt, sodass die Plattform eine Breite aufweist, die geringer als die Breite der Basis ist,
einen ersten Aufnahme-Riemen, der sich im Wesentlichen in Querrichtung von der Basis aus erstreckt, wobei der erste Aufnahme-Riemen (32) eine Länge, die sich von einem ortsfesten Ende aus, das auf einer Seite der Basis getragen wird, zu einem freien Ende hin erstreckt, und darin eine Vielzahl von Öffnungen (58, 60), die entlang der Länge beabstandet sind, aufweist;
einen zweiten Aufnahme-Riemen (34), der sich im Wesentlichen in Querrichtung von der Basis aus in einer Position erstreckt, die vom ersten Aufnahme-Riemen in Längsrichtung beabstandet ist, wobei der zweite Aufnahme-Riemen eine Länge, die sich von einem ortsfesten Ende aus, das auf einer Seite der Basis getragen wird, zu einem freien Ende hin erstreckt und darin eine Vielzahl von Öffnungen (62, 64), die entlang der Länge des zweiten Aufnahme-Riemens beabstandet sind, aufweist; und
wobei jedes der ortsfesten Enden des ersten und zweiten Aufnahme-Riemens auf einer Seite der oberen Oberfläche der Plattform getragen wird; und
einen ersten Einsteck-Riemen (36), der sich im Wesentlichen in Querrichtung von der Basis aus erstreckt, wobei der erste Einsteck-Riemen eine Länge aufweist, die sich von einem ortsfesten Ende (54) aus, das auf einer Seite der Basis getragen wird, zu einem freien Ende hin erstreckt und entlang der Länge des ersten Einsteck-Riemens eine Vielzahl von flexiblen Verschlusselementen (52) trägt, wobei die Verschlusselemente von einer solchen Größe sind, dass sie durch die Vielzahl von Öffnungen in dem ersten Aufnahme-Riemen in einem gebeugten Zustand hindurch verlaufen; und
einen zweiten Einsteck-Riemen (38), der sich im Wesentlichen in Querrichtung von der Basis aus in einer Position erstreckt, die vom ersten Einsteck-Riemen in Längsrichtung beabstandet ist, wobei der zweite Einsteck-Riemen eine Länge aufweist, die sich von einem ortsfesten Ende (56) aus, das auf einer Seite der Basis getragen wird, zu einem freien Ende hin erstreckt und entlang der Länge des zweiten Einsteck-Riemens eine Vielzahl von flexiblen Verschlusselementen (52) trägt, wobei die Verschlusselemente von einer solchen Größe sind, dass sie durch die Vielzahl von Öffnungen in dem zweiten Aufnahme-Riemen in einem gebeugten Zustand hindurch verlaufen; und
wobei die ortsfesten Enden des ersten und zweiten Einsteck-Riemens an der Basis entlang einer lateralen Seite der oberen Oberfläche der Plattform befestigt sind, (40) wobei der erste Aufnahme-Riemen und Einsteck-Riemen flexibel sind, sodass sie sich aufeinander zubewegen, und der zweite Aufnahme-Riemen und Einsteck-Riemen flexibel sind, sodass sie sich aufeinander zubewegen, wodurch die Verschlusselemente, die auf dem ersten und zweiten Einsteck-Riemen getragen werden, in ausgewählte der Öffnungen in den ersten und zweiten Aufnahme-Riemen einführbar sind beziehungsweise zum Greifen eines Schlauches und Halten des Schlauches entlang der Länge der Basis ziehbar sind,
wobei die Basis, der erste und zweite Aufnahme-Riemen und der erste und zweite Einsteck-Riemen aus einem einstückigen, weichen Elastomer mit geringem Härtegrad, aufweisend eine Härte, die geringer oder gleich 30 Shore A ist, einheitlich, integral zusammen ausgebildet sind; und
wobei der erste und zweite Aufnahme-Riemen und der erste und zweite Einsteck-Riemen an ihren ortsfesten Enden zusammengefügt werden, wodurch sie eine Mulde (77) mit einem Boden zum Empfangen und Greifen von Schläuchen bereitstellen; und wobei jedes der ortsfesten Enden des ersten und zweiten Einsteck-Riemens auf einer Seite der oberen Oberfläche der Plattform in einer Position getragen wird, die seitlich gegenüber eines des ersten oder zweiten Aufnahme-Riemens liegt, und jeder des ersten und zweiten Einsteck-Riemens ein freies Ende, das von der oberen Oberfläche der Plattform beabstandet ist, und einen gebogenen Körper zwischen dem ortsfesten Ende und dem freien Ende aufweist, um eine Schlauchgreifkrümmung zu bilden, wenn die freien Enden des ersten und zweiten Einsteck-Riemens jeweils in einer der Öffnungen in dem gegenüberliegenden des ersten oder zweiten Aufnahme-Riemens empfangen werden.

2. Schlauchhalterung gemäß Anspruch 1, wobei die obere Oberfläche der erhobenen Plattform eine kuppelförmige Konfiguration aufweist.

3. Schlauchhalterung gemäß Anspruch 2, wobei die Basis Stirnoberflächen (44, 46) aufweist, die von der oberen Oberfläche der Plattform zur unteren Oberfläche der Basis abfallend verlaufen.

4. Schlauchhalterung gemäß Anspruch 1, wobei die Hydrokolloidschicht darin eine Mündung zum Empfangen eines medizinischen Katheters angrenzend an eine Einführungsstelle aufweist.

5. Schlauchhalterung gemäß Anspruch 4, wobei die Mündung durch einen Schlitz (72) gebildet wird, der Flügel (74) definiert, die sich um den medizinischen Katheter in einer überlappenden Anordnung schlingen.

6. Schlauchhalterung gemäß Anspruch 1, wobei die Öffnungen in einem der Aufnahme-Riemen mindestens zwei Öffnungen darin umfassen, die entlang der Länge beabstandet sind, wobei eine der Öffnungen (60, 64) näher an der Basis als die andere (58, 62) der Öffnungen liegt, wobei die näher liegende Öffnung eine Höhe aufweist, die geringer als die Höhe der anderen Öffnung ist.

## Revendications

1. Un porte-tube (20) comprenant
une surface de support (66) destinée à être fixée sur la peau d'un patient incluant une couche adhésive hydrocolloïde (68) collée à un film élastomère (70) pour fournir des caractéristiques compatibles avec la peau incluant une élasticité similaire à celle de la peau humaine ;
une base (22) montée sur ladite surface de support, ladite base ayant une longueur et une largeur ; et
ladite base ayant une surface inférieure plate (42) s'étendant dans le sens de la largeur couvrant entièrement ladite largeur de ladite base et une plateforme surélevée (40) ayant une surface supérieure espacée de ladite surface inférieure et s'étendant dans le sens de la largeur ne couvrant pas entièrement une partie de ladite base de telle sorte que ladite plateforme ait une largeur inférieure à ladite largeur de ladite base,
une première sangle femelle s'étendant substantiellement transversalement à partir de ladite base, ladite première sangle femelle (32) ayant une longueur s'étendant depuis une extrémité fixe portée sur un côté de ladite base jusqu'à une extrémité libre et une pluralité d'ouvertures (58, 60) dans celle-ci espacées le long de ladite longueur ;
une deuxième sangle femelle (34) s'étendant substantiellement transversalement depuis ladite base au niveau d'une position espacée longitudinalement de ladite première sangle femelle, ladite deuxième sangle femelle ayant une longueur s'étendant depuis une extrémité fixe portée sur un côté de ladite base jusqu'à une extrémité libre et une pluralité d'ouvertures (62, 64) dans celle-ci espacées le long de ladite longueur de ladite deuxième sangle femelle ; et
chacune desdites extrémités fixes desdites première et deuxième sangles femelles étant portée sur un côté de ladite surface supérieure de la plateforme ; et
une première sangle mâle (36) s'étendant substantiellement transversalement à partir de ladite base, ladite première sangle mâle ayant une longueur s'étendant depuis une extrémité fixe (54) portée sur un côté de ladite base jusqu'à une extrémité libre et portant une pluralité d'éléments de verrouillage flexibles (52) le long de ladite longueur de ladite première sangle mâle, lesdits éléments de verrouillage étant d'une taille appropriée pour traverser ladite pluralité d'ouvertures dans ladite première sangle femelle dans un état fléchi ; et
une deuxième sangle mâle (38) s'étendant substantiellement transversalement à partir de ladite base au niveau d'une position espacée longitudinalement de ladite première sangle mâle, ladite deuxième sangle mâle ayant une longueur s'étendant depuis une extrémité fixe (56) portée sur un côté de ladite base jusqu'à une extrémité libre et portant une pluralité d'éléments de verrouillage flexibles (52) le long de ladite longueur de ladite deuxième sangle mâle, lesdits éléments de verrouillage étant d'une taille appropriée pour traverser ladite pluralité d'ouvertures dans ladite deuxième sangle femelle dans un état fléchi ; et
les extrémités fixes des première et deuxième sangles mâle étant couplées à la base le long d'un côté latéral de ladite surface supérieure de la plateforme, (40) lesdites premières sangles femelle et mâle étant flexibles pour se déplacer l'une vers l'autre et lesdites deuxièmes sangles femelle et mâle étant flexibles pour se déplacer l'une vers l'autre, grâce à quoi lesdits éléments de verrouillage portés sur lesdites première et deuxième sangles mâles peuvent être insérés dans des ouvertures choisies parmi lesdites ouvertures dans lesdites première et deuxième sangles femelles, respectivement, et tirés pour saisir un tube et maintenir le tube le long de ladite longueur de ladite base,
ladite base, lesdites première et deuxième sangles femelles et lesdites première et deuxième sangles mâles étant formées de façon unitaire en un élastomère doux, à faible duromètre, d'un seul tenant, ayant une dureté inférieure ou égale à 30 Shore A ; et
lesdites première et deuxième sangles femelles et lesdites première et deuxième sangles mâles se rejoignant mutuellement au niveau de leurs extrémités fixes, fournissant un bac (77) doté d'un fond pour recevoir et saisir des tubes ; et chacune desdites extrémités fixes desdites première et deuxième sangles mâles étant portée sur un côté de ladite surface supérieure de la plateforme au niveau d'une position latéralement opposée à l'une desdites première ou deuxième sangles femelles et chacune desdites première et deuxième sangles mâles ayant une extrémité libre espacée de ladite surface supérieure de la plateforme et un corps arqué entre ladite extrémité fixe et ladite extrémité libre pour former une courbure de saisie de tube lorsque lesdites extrémités libres desdites première et deuxième sangles mâles sont chacune reçues dans l'une desdites ouvertures dans la sangle opposée parmi lesdites première et deuxièmes sangles femelles.

2. Un porte-tube tel qu'énoncé dans la revendication 1 dans lequel ladite surface supérieure de ladite plateforme surélevée a une configuration en dôme.

3. Un porte-tube tel qu'énoncé dans la revendication 2 dans lequel ladite base a des surfaces d'extrémité (44, 46) inclinées depuis ladite surface supérieure de ladite plateforme jusqu'à ladite surface inférieure de ladite base.

4. Un porte-tube tel qu'énoncé dans la revendication 1 dans lequel ladite couche hydrocolloïde a un orifice dans celle-ci pour recevoir un cathéter médical adjacent à un site d'insertion.

5. Un porte-tube tel qu'énoncé dans la revendication 4 dans lequel ledit orifice est formé par une fente (72) définissant des rabats (74) destinés à s'enrouler autour du cathéter médical selon un arrangement de chevauchement.

6. Un porte-tube tel qu'énoncé dans la revendication 1 dans lequel lesdites ouvertures dans l'une desdites sangles femelles incluent au moins deux ouvertures espacées dans celle-ci le long de ladite longueur, l'une desdites ouvertures (60, 64) étant plus proche de ladite base que l'autre ouverture (58, 62) parmi lesdites ouvertures, ladite ouverture plus proche ayant une hauteur inférieure à la hauteur de ladite autre ouverture.
